# NEUE EUROPÄISCHE PATENTSCHRIFT

(11) **EP 1 701 890 B3**
(45) Veröffentlichungstag dieser Patentschrift: **23.08.2017**
(45) Hinweis auf die Patenterteilung: 26.08.2009
(21) Anmeldenummer: 04804316.0
(22) Anmeldetag: 27.12.2004
(51) Int. Cl.: B65D 51/24, A61M 15/00

(54) **VERFAHREN UND VORRICHTUNG ZUM ERSTMALIGEN FÜLLEN DER DOSIERKAMMER EINES INHALATORS**
METHOD AND DEVICE FOR FILLING THE DOSING CHAMBER OF AN INHALER FOR THE FIRST TIME
PROCEDE ET DISPOSITIF POUR LE REMPLISSAGE INITIAL DE LA CHAMBRE DE DOSAGE D'UN INHALATEUR

(30) Priorität: 29.12.2003 DE 10361735
(43) Veröffentlichungstag der Anmeldung: 20.09.2006
(73) Patentinhaber: Boehringer Ingelheim International GmbH, 55216 Ingelheim am Rhein (DE)
(72) Erfinder: GESER, Johannes, 55218 INGELHEIM (DE); BOECK, Georg, 88471 LAUPHEIM (DE); SPALLEK, Michael, 55218 Ingelheim am Rhein (DE)
(74) Vertreter: Hammann, Heinz
(86) Internationale Anmeldenummer: PCT/EP2004/014726
(87) Internationale Veröffentlichungsnummer: WO 2005/063591

(56) Entgegenhaltungen:
- WO-A-00/49988
- GB-A- 698 130
- US-A- 3 215 171

## Beschreibung

Die vorliegende Erfindung betrifft Inhalatoren für medizinische Zwecke gemäß dem Oberbegriff des Anspruchs 8, die eine vorgegebene Menge einer bevorzugt pharmazeutischen Flüssigkeit über einen längeren Zeitraum als "weiche" Aerosolnebel zur Inhalation ausbringen ("soft mist^{™} inhaler" oder kurz SMI). Um ein solches Gerät handelt es sich beispielsweise bei dem Inhalator des Typs Respimat^{®}, der in der WO 97/12687 näher beschrieben wird. Bei dieser Art, wird der Inhalator mit einer eine größere Menge der Wirkstoffformulierung enthaltenden Kartusche bestückt. Die vorliegende Erfindung betriff auch eine Weiterentwicklung des Respimat^{®} -Systems, bei welchem das Bestücken des Geräts mit der Kartusche im Hinblick auf einen schnellen ersten Sprüheinsatz verbessert wird, gemäß den im Oberbegriff des Anspruchs 1 genannten Merkmalen.

### Stand der Technik

Die handlichen Inhalatoren des Respimat^{®} -Typs oder Inhalatoren vom Typ Respimat^{®} Soft^{®} Mist™ Inhaler (SMI), bei welchen eine kleine Menge einerwässrigen Formulierung ohne Hilfe von Treibgasen im Bereich von wenigen Mikrolitem in einen Aerosolnebel zerstäubt wird, sind eine der jüngsten innovativen Entwicklungen auf dem Gebietder medizinischen Vernebelungstechnik. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführtwerden, so dass es damit auch für den regelmäßigen täglichen Gebrauch in bequemer Weise für den Patienten unabhängig vom Ort zur Verfügung steht.

Die technischen Grundlagen dieser Inhalatoren werden z.B. in der WO 91/14468 oder der WO 97/12687, dort besonders die Figuren 6a und 6b, offenbart. Bei diesen Inhalatoren wird die zu vernebelnde Menge derflüssigen Arzneimittelformulierung mittels hohen Drucks von bis zu 50 MPa (500 bar) durch eine Mikrodüse mit bevorzugtzwei Düsenausgängen gepresst und dabei in das lungengängiges Aerosol überführt. Auf die genannten Referenzen wird im Rahmen der vorliegenden Erfindungsbeschreibung ausdrücklich in Gänze Bezug genommen. Dem Respimat^{®}-Prinzip liegen dabei zwei getrennte bauliche Einheiten zugrunde: Zum einen der Inhalator, der die gesamte Mechanik zur Erzeugung des Aerosols enthält und zum anderen eine davon baulich getrennte Kartusche, die die Arzneistoffformulierung enthält.

Zum Gebrauch wird die Kartusche auf eine in dem Inhalator ausgebildete Kanüle aufgesteckt. Durch diese Kanüle wird Flüssigkeit aus der Kartusche in eine Druck- und Dosierkammer gefördert und von dort aus unter Anwendung von Druck durch eine Mikrodüse gepresst. Die Kartusche besteht aus einem mit der Flüssigkeit gefüllten Behälter und einer Verschlusskappe für denselben.

Im wesentlichen besteht ein Zerstäuber vom Respimat^{®} -Typ aus einem kopfseitigen Gehäuseoberteil, einem Gehäuseunterteil, welches gegenüber dem Gehäuseoberteil drehbar gelagerten ist und die Bodenseite definiert, einem Pumpengehäuse, der Düse, einem Sperrspannwerk, einem Federgehäuse, einer Feder und dem Vorratsbehälter.

Das Pumpengehäuse befindet sich im Gehäuseoberteil. An dem kopfseitigen Ende des Pumpengehäuse befindet sich der Düsenkörper mit der Düse bzw. die Düsenanordnung. Darunter befindet sich eine Druckkammer, die Teil eines Zentralrohrs in Form einer zylindrischen Bohrung sein kann. Unterhalb der Druckkammer befindet sich das obere Ende eine Kanüle in Form eines Hohlkolbens, die teilweise in das Zentralrohr hineinragt und dort axial hubartig hin und herbewegtwerden kann. DerHohlkolben ist außerhalb des Zentralrohrsfest mit einem Abtriebsflansch verbunden. Der Abtriebsflansch sitzt auf dem kopfseitigen Ende einer Feder (Schraubfeder) und wird über diese bewegt. Die Schraubfeder befindet sich in einem Federgehäuse, das am Gehäuseoberteil mittels eines Drehlagers drehbar gelagert ist und über ein Sperrspannwerk gespannt und entspannt werden kann. Alle die in diesem Abschnitt aufgeführten Bauelemente befinden sich im Gehäuseoberteil. Der Hohlkolben reicht dabei mit seinem bodenseitigen, unteren Ende bis in den durch die Schraubfeder definierten inneren Hohlraum. Dieser Hohlraum ist nach unten hin offen. Nach oben hin kann er vom dem Abtriebsflansch begrenzt sein. Die Arzneimittelkartusche wird vom bodenseitigen Ende in diesen Hohlraum der Schraubfeder eingeführt und auf die Kanüle gesteckt. Das Gehäuseunterteil wird dann in axialer Richtung über das das Federgehäuse aufgesteckt. Das System aus Hohlkolben, Zentralrohr, Druckkammer und Düse stellt ein System zum Leiten einer Flüssigkeit dar. Die Verbindungsstellen zwischen den einzelnen Bauteilen sind nach Außen hin dicht. Das Flüssigkeitsleitsystem besitzt nur zwei Öffnungen, die untere Öffnung des Hohlkolbens und die Düsenöffnung. Die eine Öffnung dient zur Aufnahme einer Flüssigkeit, die andere, die Düsenöffnung, zur Abgabe derselben.

Als Düsen werden spezielle Düsen verwendet, wie sie beispielsweise die WO 94/07607 oder die WO 99/16530 beschreiben. Auf beide wird hiermit ausdrücklich Bezug genommen.
Die Düse im Düsenkörper ist bevorzugt mikrostrukturiert, d.h. durch Mikrotechnik hergestellt. Mikrostrukturierte Düsenkörper sind beispielsweise in der WO-94/07607 offenbart; auf diese Schrift wird hiermit inhaltlich Bezug genommen, insbesondere auf Figur 1 und deren Beschreibung.
Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt.
Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen mit einem Winkel von 20 Grad bis 180 Grad gegeneinander geneigt sein, bevorzugt wird ein Winkel von 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.
Die Strahlrichtungen treffen sich dementsprechend in der Umgebung der Düsenöffnungen.

Für die Vernebelung trifft die flüssige Arzneimittelzubereitung mit einem Eingangsdruck von bis zu 60 M Pa (600 bar), bevorzugt 20 bis 30 M Pa (200 bis 300 bar) auf den Düsenkörper und wird letztlich so über die Düsenöffnungen in ein inhalierbares Aerosol zerstäubt. Die bevorzugten Teilchengrößen des Aerosols liegen bei bis zu 20 Mikrometern, bevorzugt 3 bis 10 Mikrometern.

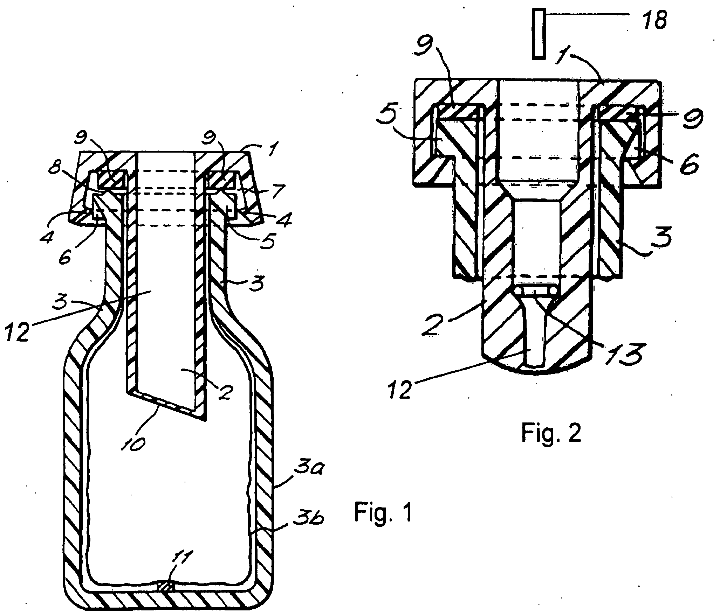

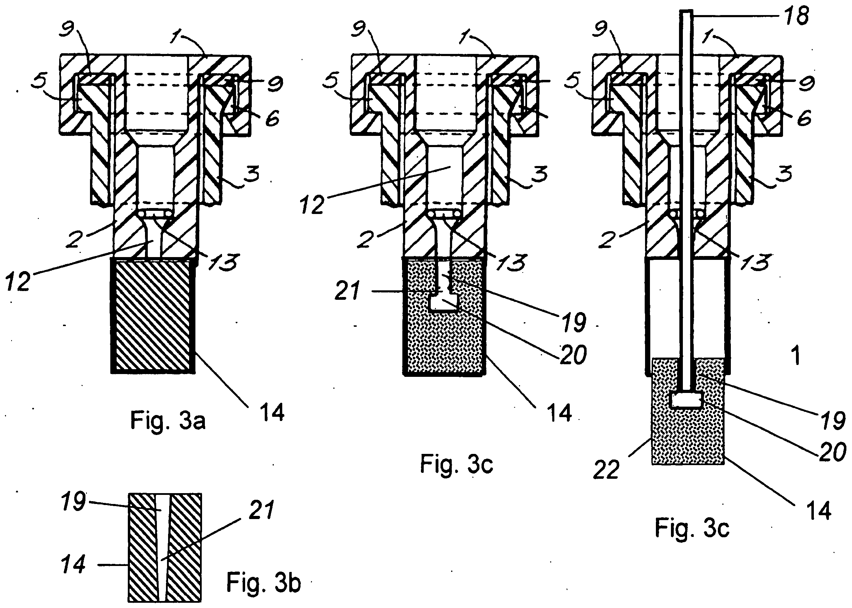

Der Hohlkolben mit Ventilkörper entspricht einer in der WO 97/12687 offenbarten Vorrichtungen. Er ragt teilweise in den Zylinder des Pumpengehäuses hinein und ist im Zylinder axial verschiebbar angeordnet. Der Ventilkörper ist bevorzugt an dem Ende des Hohlkolbens angebracht, das dem Düsenkörper zugewandt ist.
Insbesondere wird auf die Figuren 1-4 - insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 MPa (etwa 50 bis 600 bar), bevorzugt 10 bis 60 MPa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 15 Mikroliter pro Hub.

Das Sperrspannwerk enthält die Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- odermehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseoberteil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Bei dem Vorratsbehälter (Kartusche) handelt es sich bevorzugt um einen Behälter mit einem Flansch oder einer Verschlusskappe, über den (die) der Hohlkolben des Inhalators in das Innere eingeführt werden kann. Der Flansch oder die Verschlusskappe enthält dabei eine Führungspassage fürden Hohlkolben mit wenigstens einer Dichtungsstelle, die verhindert, dass außen am Holkolben entlang Luft in das Innere des Behälters gelangen kann oder über diesen Weg Flüssigkeit aus dem Inneren des Behälters austritt. Der Flansch oder die Verschlusskappe können derart sein, dass sie lösbar oder unlösbar mit dem Abtriebsflansch des Inhalators verbunden werden können. Bevorzugt ist dieser Behälter als kollabierbaren Behälter ausgebildet, der dabei bevorzugt von einemfesten, steifen zweiten Behälterumgeben sein kann, der den kollabierbaren ersten Behälter u.a. vor Beschädigung schützt. Geeignete Behälter werden in der der EP 0775076 oder der WO 99/43571 beschrieben. Aber auch andere geeignete nicht kollabierbare Behälter können verwendet werden. Der Vorratsbehälter stellt vor dem Aufstecken auf den Hohlkolben ein in sich abgeschlossenes System dar, an welchem sich keine Vorrichtungen zum Beaufschlagen mit Druck befinden.

Vor der ersten Benutzung muss die noch verschlossenen Kartusche (der Behälter) auf die Kanüle des Inhalators aufgesteckt werden. Zum erstmaligen vollständigen Füllen des Bereichs vom Holkolben bis zur Düse mit Flüssigkeit soll der aus dem Stand der Technik bekannte Inhalator mehrmals gespannt und ausgelöst werden.

### Beschreibung der Erfindung

Es ist eine Aufgabe dervorliegenden Erfindung einen Inhalator vom Respimat^{®} -Typ zu schaffen, der nach dem ersten Einsetzen der Kartusche schneller in Betrieb genommen werden kann, als das aus dem Stand der Technik bekannte Gerät.

Eine weitere Aufgabe der vorliegende Erfindung besteht darin, die Schritte zur ersten Inbetriebnahme eines Inhalatoren vom Respimat^{®}-Typ zu verkürzen.

Eine weitere Aufgabe der vorliegenden Erfindung besteht darin, die Schritte zum ersten Füllen eines Inhalatoren vom Respimat^{®} -Typ mit Flüssigkeit zu automatisieren.
- Fig. 1: zeigt eine Kartusche für einen Inhalator;
- Fig. 2: zeigt eine bevorzugte Ausführungsform einer Verschlusskappe für die Verwendung im Inhalator;
- Fig. 3(a): zeigt eine Ausführungsform des Inhalators;
- Fig. 3(b): zeigt eine bevorzugte Ausführungsform des Inhalators;
- Figuren 3c: zeigen eine weitere Ausführungsform des in Fig. 3b dargestellten Inhalators, wobei die Kapillaren (22) als Mikrokapillaren ausgebildet sind. Die Figuren 3c zeigen unterschiedliche Vergrößerungen;
- Fig. 4a und 4b: zeigen eine weitere Ausführungsform des in Fig. 3b dargestellten Inhalators, mit einem Eintauchstutzen aus 2 oder mehr teleskopartig ineinander stechenden Hülsen (15, 15', 15°);

- Fig. 5a Fig. 5b: zeigen eine Ausführungsform des Inhalators mit einem Eintauchstutzen, welcher im unteren Bereich ziehharmonika-ähnlich zusammengefaltet ist.

### Detaillierte Beschreibung der Erfindung

Erfindungsgemäß liegt die Aufgabe zugrunde, den Vorgang zum Auffüllen des Totvolumens im Inhalator zeitlich zu beschleunigen und zu automatisieren. Dabei wird als Totvolumen das Volumen verstanden, welches durch das Innere der Kanüle oberhalb des Flüssigkeitsspiegels, dem Inneren des Ventils, den darüber liegenden Bereich des Zylinders inklusive der Druckkammer und dem Düseninnenraum geschaffen wird abzüglich des Anteils an Volumen, den der Bereich des Hohlkolbens einnimmt. D.h. nicht berücksichtigt ist der Teil des Kanülenvolumens, der nach vollendetem Aufsteckvorgang der in der Regel zu wenigstens 90 Volumen% gefüllten Kartusche in die Flüssigkeit hineinragt. Das System aus Kanüle, Zylinder, Druckkammer, Düsewirdfolgend auch als Flüssigkeitsleitsystem bezeichnet. Damit entspricht das Totvolumen dem inneren Volumen des Flüssigkeitsleitsystems bei entspannter Feder abzüglich dem sich von allein durch das Prinzip der kommunizierenden Röhren füllenden Teils, wenn die Kartusche auf die Kanüle gesteckt wird. Ebenfalls nicht inbegriffen ist das durch den Zerstäuber auszubringende Volumen. Dieses Volumen wird Füllvolumen genannt und wird erzeugt, wenn die Feder des Gerätes gespannt wird und sich der Kolben aus dem Zentralrohr hinaus bewegt ohne ihn jedoch zu verlassen. Die Differenz der beiden Volumina entspricht dabei in etwa der Menge an Flüssigkeit die vernebelt werden soll (Ausbringvolumen).

Im Detail lässt sich der bevorzugte Vernebler wie folgt beschreiben. In einem zylindrischen Gehäuseoberteil befindet sich ein Pumpengehäuse. An dessen Ende ist ein Halter für die Zerstäuberdüse angebracht. In dem Halter befindet sich der Düsenkörper und ggf. ein oder
mehrere Filter. Die Düse befindet sich am oberen Ende eines Zylinderrohrs, das im Pumpengehäuse ausgebildet ist. Der in einem Abtriebsflansch des Sperrspannwerkes befestigte Hohlkolben ragt teilweise durch das untere Ende des Zylinderrohrs hinein. An seinem Ende trägt der Hohlkolben einen Ventilkörper. Der Hohlkolben ist mittels einer Dichtung nach Außen abgedichtet. Innerhalb des Gehäuseoberteils befindet sich ein erster Anschlag, an dem der Abtriebsflansch bei entspannter Feder anliegt. Am Abtriebsflansch befindet sich ein zweiter Anschlag, an dem der Abtriebsflansch bei gespannter Feder anliegt. Nach dem Spannen der Feder schiebt sich ein Sperrglied zwischen den zweiten Anschlag und eine Abstützung im Gehäuseoberteil. Eine Auslösetaste steht mit dem Sperrglied in Verbindung. Das Gehäuseoberteil endet in einem Mundstück und ist mit der aufsteckbaren Schutzkappe verschlossen.

Ein zylindrisches Federgehäuse mit Druckfeder ist mittels Schnappnasen und Drehlager am Gehäuseoberteil drehbar gelagert. Über das Federgehäuse ist das zylindrisches Gehäuseunterteil geschoben. Innerhalb des Federgehäuses befindet sich der austauschbare Vorratsbehälter für das zu zerstäubende Fluid. Der Vorratsbehälter ist mit einem Stopfen verschlossen, durch den der Hohlkolben in den Vorratsbehälter hineinragt und mit seinem Ende in das Fluid (Vorrat an Wirkstofflösung) eintaucht.

Um die erfindungsgemäße Aufgabe zu lösen, wird vorgeschlagen, einen im Vorratsgefäß (Behälter) spontan aufgebauten oder darin vorhandenen Überdruck mit dem Einbringen des Vorratsgefäßes in den Inhalator unter Verdrängung von Flüssigkeit aus dem Behälter durch das Flüssigkeitsleitsystem aus Hohlkolben, Zylinder, Druckkammer und Düse zu entspannen, so dass das Totvolumen des Flüssigkeitsleitsystems mit Flüssigkeit gefüllt und die Düse an den Flüssigkeitsvorrat luftfrei angebunden ist.

Erfindungsgemäß soll der Überdruck ausreichen, um auf der einen Seite das Totvolumen mehr als vollständig mit Flüssigkeit aufzufüllen. Auf der anderen Seite soll der Druck nur so hoch sein, dass bevorzugt wenigerals 100 Mikroliter den Inhalator aufgrund der Entspannung durch die Düse verlassen. Dabei ist wichtig, dass wenigstens ein-einhalb mal mehr Flüssigkeit durch das Flüssigkeitsleitsystem gepresst wird, als es dem Totvolumen des Flüssigkeitsleitsystems entspricht. Dadurch werden Toleranzen ausgeglichen, die sich aufgrund der Elastizität des Vorratsgefäßes ergeben können.

In einer ersten Ausführungsform der Erfindung wird der Überdruck in dem Behälter spontan durch Aufstecken des Behälters auf den Hohlkolben des entspannten Inhalators erzeugt. Dabei wird zumindest der Bereich der Kanüle des Inhalators, der in den Behälter hineinreicht, anders ausgestaltet, als die aus dem Stand der Technik bekannte Ausführungsform. Erfindungsgemäß soll der Bereich der Kanüle des Inhalators, der in den Behälter hineinreicht, so gestaltet sein, dass dieser Bereich wenigstens ein-einhalb mal soviel, bevorzugt doppelt soviel Flüssigkeit verdrängt, wie es dem ersten Totvolumen entspricht. Diese Verdrängung erfolgt durch einen Verdrängungskörper, der nachfolgend näher beschrieben wird. Durch diese Maßnahme wird der Druck, der durch das Aufstecken des Vorratsbehälters auf die Kanüle im Inneren des Behälters erzeugt wird erhöht, mit der Folge, dass die Flüssigkeit mit höherem Druck und damit schneller durch den Überdruck im Inneren des Behälters durch die Kanüle in Richtung Düse gedrängt wird.

Das Totvolumen des Flüssigkeitsleitsystems des bekannten Systems beträgt ca. 17 Mikroliter, das sich aus ca. 10 Mikroliter Totvolumen im Zentralrohr bei entspannter Feder inklusive des Totraums der Druckkammer, 7 MikroliterTotvlumen in der Kapillare (das ist der Anteil des Kapillarvolumens, das sich bei aufgesteckter vollständig gefüllter Kartusche oberhalb des Flüssigkeitspegels befindet) und ca.100 Nanolitern Totraum der Düse zusammensetzt. Dieses Volumen muss dann von dem Bereich der Kanüle, welcher beim Aufstecken der Kartusche auf die Kanüle in die Flüssigkeit eintaucht verdrängt werden. Bei einem Außendurchmesser der Kanüle von 1,5 mm und einer Wandstärke von 1,1 mm, muss der Teil der Kanüle, der in die Flüssigkeiteintaucht, ca.10,8mm betragen, um ein Volumen von 18 Mikrolitem zu verdrängen.

Es hat sich aber gezeigt, dass diese Maße das Problem nicht lösen, da der elastische Behälter den erzeugten Überdruck teilweise kompensiert.

Die erfindungsgemäße Aufgabe wird in diesem Beispiel nur dann vollständig gelöst, wenn das Verdrängungsvolumen des Teiles des Hohlkolbens, der in das Innere des Behälters hineinragt, wenigstens 23 Mikroliter, stärker bevorzugt wenigstens 34 Mikroliter beträgt.

Um bei gleich bleibenden Innen- und Außendurchmesser des Hohlkolbens das Verdrängungsvolumen auf die oben genannten bevorzugten Werte zu erhöhen, muss die in das Innere des Behälters hineinragende Länge des Hohlkolbens auf wenigstens 13,8 mm erhöht werden, bevorzugt auf wenigstens 20,4 mm.

In einer anderen Ausführungsform wird der Außendurchmesser der Kanüle erhöht, bei gleich bleibenden Innendurchmesser und gleich bleibender Eintauchtiefe in das Behälterinnere. Zielführend ist in diesem Fall ein Außendurchmesser von wenigstens 1,7 mm, bevorzugtwenigstens 2 mm. Diese Maßnahme hat den Vorteil, dass durch die breite effektive Stempelfläche der Kanüle der Anfangsdruck im inneren des Behälters schneller aufgebaut wird, so dass der Druck auf die Flüssigkeit durch die Kanüle zu entweichen zunächst stärker erhöht wird, als durch Verlängerung der Kolbens.

Bei einer weiteren Ausführungsform kann der Kolben sowohl verlängert, als auch dessen Außendurchmesser vergrößert werden. Dabei kann auch nur der Bereich des Kapillare, der in die Flüssigkeit eintaucht entsprechend gestaltet werden, z. B. einen größeren Außendurchmesser aufweisen, als der restliche Bereich der Kanüle.

In allen Fällen soll bevorzugt die Länge des Holkolbens von 44,2 mm außerhalb des Behälterinneren erhalten bleiben.

In einer anderen Ausführungsform wird der Behälter selbst beim Abfüllen der Arzneimittelformulierung mit Druck beaufschlagt. Dies kann z.B. dadurch geschehen, dass der Behälter bei tiefen Temperaturen, z.B. von 4°C bis 10°C abgefüllt und versiegelt wird, (Kaltabfüllung). Bei Erwärmung auf Raumtemperatur wird dann der entsprechende Überdruck durch Ausdehnung der Flüssigkeit erzeugt.

In wieder einer anderen Ausführungsform wird beim Abfüllen der Arzneimittelformulierung in dem Behälter ein Überdruck erzeugt, in dem die Arzneimittelformulierung unter Überdruckatmosphäre abgefüllt wird und im Inneren des Behälters eine Luftblase in der entsprechenden Größenordnung belassen wird. Anschließend wird der Behälter versiegelt.
Bei diesem Verfahren wird die Luftblase beim Abfüllen komprimiert. Beim Anstechen des Behälters mit der Kanüle entspannt sich die Luftblase und drückt dabei die Flüssigkeit durch die Kanüle. Entsprechend den obigen Ausführungen beträgt die Volumendifferenz zwischen komprimierter und entspannter Luftblase bevorzugt wenigstens 23 Mikroliter, stärker bevorzugt wenigstens 34 Mikroliter. Bevorzugt wird dabei eine Luftblase von weniger als 100 Mikrolitern im Behälter belassen. Auch bei dieser Ausführungsform muss die Kanüle des Inhalators in die Flüssigkeit eintauchen.

Weitergehende Einzelheiten zum Abfüllvorgang können dem genannten Stand derTechnikentnommen werden.

Durch die genannten Maßnahmen wird im Inneren des Behälters ein Überdruck von bevorzugt mehr als 1 mbar aufgebaut, besonders bevorzugt von mehr als 500Pa (5 mbar). Der maximal aufgebaute Druck soll dabei 5 kPa (50 mbar) nicht überschreiten.

In einer anderen Ausführungsform wird nicht der Inhalator, sondern die dazu passende Kartusche, d.h. das Vorratssystem für die Flüssigkeit aus Behälter und Verschluss, physikalisch verändert, indem eine Verdrängungseinrichtung ausgebildet wird, die beim Aufstecken der Kartusche auf die Kanüle des Inhalators in das Behälterinnere hinein geschoben wird und dabei einen Teil der Flüssigkeit durch das Flüssigkeitsleitsystem verdrängt. Im Folgenden werden derartige Ausführungsformen durch die Figuren 1 bis 5 näher erläutert. Die Darstellungen sind nicht maßstabsgetreu und haben teilweise skizzenhaften Charakter.

Eine typische Kartusche wird z.B. durch Fig. 1 beschrieben. Der Verschluß (1) weist eine Einrichtung (2) in Form eines Stutzens auf. Der Stutzen kann ggf. während des Verschließvorganges ein Teil des Inhalts aus dem Behälter (3) verdrängen. Der Eintauchstutzen (2) weist seinerseits einen Durchgang oder Führung (12) für die Kanüle (18) des Inhalators auf. Der Stutzen (2) ist zunächst bodenseitig geschlossen. Der Eintauchstuzen (2) verdrängt beim Aufsetzen der Verschlusskappe Flüssigkeit aus dem Behälter und gewährleistet dadurch, dass der Behälter nach Verschließen zu wenigstens 90, bevorzugt 95 Volumen % gefüllt ist. Die Verschlusskappe weist ferner einen innenliegenden umlaufender Wulst (4) (Crimpkante) auf, der am unteren Rand der Verschlußkappe (1) in der Verschlußposition unterhalb eines an der Außenseite des Behälterhalses umlaufenden zylindrischen Ringes (5) einrastet. Während die Verschlußkappe (1) aufgeschoben wird, wird der Rand der Verschlußkappe gedehnt und der Wulst (4) liegt abdichtend auf dem Ring (5) auf, so daß nur durch eine oder mehrere Entlüftungsöffnungen (6) eine Verbindung vom Kappeninnenraum (7) nach außen besteht. Die Entlüftungsöffnung(en) ist (sind) z.B. im äußeren Bereich des Rings (5) angeordnet. In der Verschlußposition wird der Zwischenraum zwischen dem flachen Teil der Verschlußkappe (1) und dem oberen Rand des Behälterhalses, der ggf. zur besseren Abdichtung mit einer umlaufenden Rippe (8) versehen ist, durch einen Dichtungsring (9) ausgefüllt und damit der Innenraum des Behälters (3) zuverlässig gegenüber dem Kappeninnenraum (7), der den Dichtungsring (9) und den Hals des Behälters (3) umgibt, abgedichtet. Der Innendurchmesser des Dichtungsrings (9) wird zweckmäßig so gewählt, daß er an der Vorrichtung (2) eng anliegt. Die Entlüftungsöffnung(en) (6) kann (können) sich auch an anderen Stellen der Kappenaußenseite befinden, etwa seitlich in dem zylindrischen Teil der Kappe. Der Eintauchstutzen weist einen durchstechbaren Boden (10) auf.

In einer bevorzugten Ausführungsform besteht der Behälter (3) aus einem formstabilen Außenbehälter und einem leicht verformbaren Innenbeutel (3b), der bei Flüssigkeitsentnahme in sich zusammenfällt. Solche Behälter sind beispielsweise in der Europäischen Patentschrift 532 873 beschrieben, auf die hiermit inhaltlich Bezug genommen wird. Die Vorrichtung (11) dient zur Befestigung des verformbaren Innenbeutels (3b) an der dem Beutel (3b) zugewandten Innenwand des äußeren starren Behälters (3a).

Die Figur 2 zeigt eine bevorzugte Ausführungsform der erfindungsgemäßen Verschlußkappe, bei der der Innenraum des Stutzens eine spezielle Führung (12) für eine Kanüle zur Flüssigkeitsentnahme aufweist. Im vorliegenden Fall sind die Entlüftungsöffnungen (6) am oberen Teil des Behälters (3) angebracht. Die Entlüftungsöffnungen können alternativ auch an der Verschlußkappe angeordnet sein. Gegebenenfalls kann die Führung (12) als Presspassung für die Kanüle (18) ausgebildet sein oder in ihr kann eine O-Ringdichtung (13) angebracht sein.

Figur 3a zeigt eine Ausführungsform der Erfindung, bei der unterhalb der Führung (12) ein Holraum in dem Eintauchstutzen (2) ausgebildet ist, in dem sich ein Verdrängungskörper (14) in Form z.B. eines Stopfens, Zylinders, Korken u.ä. befindet, der beim Durchführen der Kanüle durch die Führung (12) wenigstens teilweise in den Behälter (3) hinein geschoben wird und dadurch hilft, im Inneren des Behälters den gewünschten Überdruck aufzubauen. Ein solcher Verdrängungskörper kann sich an jeder Stelle in der Führung (12) befinden. Die Form des Verdrängungskörpers ist bevorzugt zylindrisch. Der Verdrängungskörper besteht bevorzugt aus einem Kunststoff wie Polyethylen, Polypropylen etc. Der Verdrängungskörper kann an seinem zum Kopfende des Verschlusses gerichteten Seite eine Aussparung aufweisen, in die die Kanüle eingreifen kann.

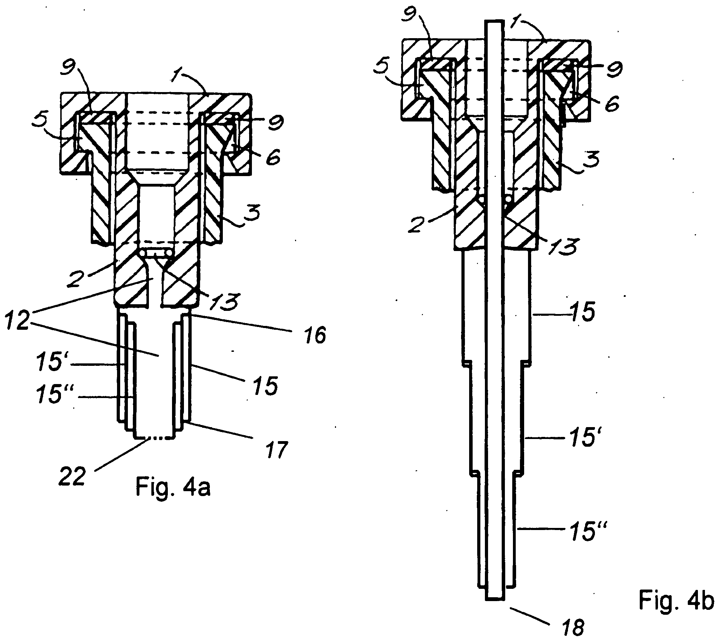

BevorzugtistderVerdrängungskörperalStempel ausgebildet, der nur teilweise aus der Führung (12) austreten kann. In diesem Fall müssen wenigstens ein Teil der Wandung des Verdrängungskörpers (14) und der.Wandung der Führung (12) flüssigkeitsdichtend wechselwirken. Damit der Verdrängungskörper (14) die Führung (12) nicht verlassen kann, können am oberen Ende des Verdrängungskörpers (14) Stoppmittel z.B. in Form einer umlaufenden Kante ausgebildet sein, die mit Stoppmitteln - z.B. ebenfalls eine umlaufende Kante - am untern Ende der Führung (12) wechselwirken. Hierdurch wird verhindert, dass Flüssigkeit in den Raum einströmt, derzuvorvon dem Verdrängungskörper(14) ausgefüllt war, so dass auf diese Weise keine Kompensation des Drucks möglich ist. Alternativ dazu können am Verdrängungskörper (14) Führungskanäle ausgebildet sein, die mit komplementären Mitteln an der Führung wechselwirken, wobei die Führungskanäle kopfseitig am Verdrängungskörper (14) nicht mehr ausgebildet sind. Weitere Stoppmittel für ein solches System werden u.a. unter Figur 4 oder im Stand der Technik beschrieben.

In einer bevorzugten Ausführungsform kann der Verdrängungskörper eine Bohrung (19) aufweisen (Fig. 3b), in die die Kanüle kraftschlüssig eingreift. Auf diese Weise wird verhindert, dass der Verdrängungskörper nach verlassen der Führung (12) in den Behälter fallen kann. Bevorzugt kann die Bohrung eine Presspassung oder Engstelle (21) aufweisen, in die Kanüle eingreift. Die Bohrung ist dabei derart gestaltet, dass die Kanüle (18) mit der Flüssigkeit im Behälter im Kontakt steht. Dafür kann die Bohrung linear durchgängig sein. In einer Variation dieser Ausführungsform istdie Bohrung zur kraftschlüssigen Aufnahme der Kanüle nicht durchgängig und so ausgebildet, dass die Kapillare nur teilweise in die Bohrung geschoben werden kann. Dadurch bildet sich unterhalb der Kapillare ein Holraum (20). Dies Dafür kann beispielsweise in der Bohrung eine Engstelle (21) ausgebildet sein, die ein weiteres Vordringen der Kapillare verhindert. Der Verdrängungskörper weist dann weiter Kapillaren (22) auf, die vom Äußeren des Verdrängungskörpers bis zu dem Holraum führen. Sind diese weiteren Kapillaren des Verdrängungskörpers als Mikrokapillaren ausgebildet, wird ständig Flüssigkeit von Außen in den Hohlraum transportiert, wodurch gewährleistet wird, dass die Kanüle des Inhalators auch im Laufe des Entleerens des Behälters immer mit Flüssigkeit versorgt wird. Eine solche Ausführungsform ist nicht maßstabsgetreu in den Figuren 3c und 3d dargestellt.

BevorzugtistderVerdrängungskörperin dieser Variante als einstückiges, von Kapillaren durchzogenes, offenporig poröses Speichermedium für Flüssigkeit ausgebildet ist. D.h. der Verdrängungskörper erfüllt gleichzeitig die Aufgabe eines Schwamms, der Flüssigkeit in sein Inneres leitet. Dabei kann der Verdrängungskörper ein formstabiler Körper sein mit einer flüssigkeitsdurchlässigen Wand, gefüllt mit gesintertem oder nicht gesintertem Pulver ist, oder ein Gewebe oder ein Gewirk oder ein Vlies oder ein Bausch aus Fasern. Er kann aus Kunststoff, Keramik, Glas, Metall oder aus einem Naturstoff bestehen.

Figur 4a und 4b zeigen eine anderen Ausführungsform, bei der der Eintauchstutzen aus wenigstens zwei oder mehrteleskopartig ineinander steckenden Hülsen (15, 15', 15") aufgebaut ist. Die jeweils inneren Hülsen weisen an ihrem oberen Ende außensitzende Stoppmittel (16) auf, die mit korrespondierenden nach Innen gerichtetete Stoppmittel am unteren Ende (17) der jeweils äußeren Hülse wechselwirken und so verhindern, dass eine innere Hülse gänzlich durch eine äußere Hülse geschoben werden kann. Die Stoppmittel sind bevorzugt Kanten. Bevorzugt sitzen die einzelnen zylinderartigen Hülsen abdichtend ineinander. Der Boden der innersten Hülse ist zunächst geschlossen, so dass die Kanüle (18) die noch ineinander geschobenen Hülsen (Figur 4a) beim Aufstecken des Behälters auseinander schiebt, bevor sie den Boden (22) durchsticht (Figur 4b). Auf dieses Weise wird der Eintauchstutzen während des Aufschiebens des Behälters auf die Kanüle (18) verlängert und wirkt so selbst als Verdrängungskörper, der in dem mit Flüssigkeit befüllten Behälterinneren einen Überdruck aufbaut.

Der innere Durchmesser der innersten Hülse kann dabei als Presspassung für die Hülse aufgebaut sein, oder es findet sich hier beispielsweise ein O-förmiger Dichtungsring, um die Kanüle (18) nach Außen hin abzudichten.

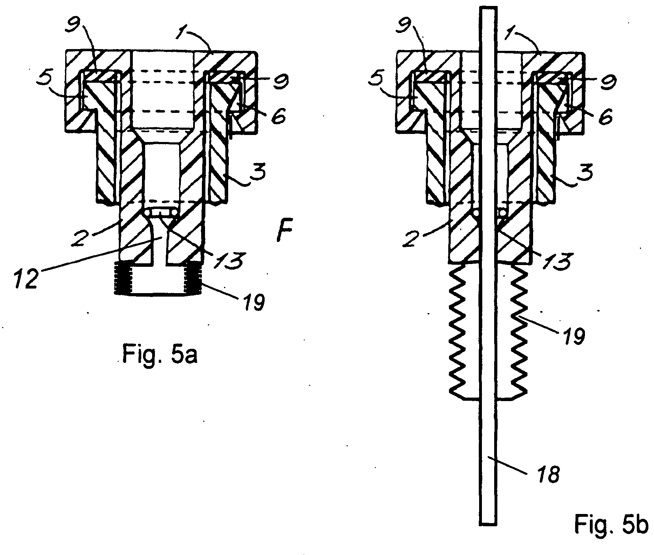

In der äquivalenten Ausführungsform nach den Figuren 5a und 5b ist der untere Bereich des Eintauchstutzens (19) zieharmonika-ähnlich zusammengefaltet (Faltenbalg) (Figur 5a). Die Führung (12) reicht dabei vom Kopfbereich der Verschlusskappe bis vor das gefaltete Ende des Eintauchstutzens. Der Bodenbereich des Eintauchstutzens ist wie bei allen Ausführungsformen so ausgebildet, dass er von der Kanüle durchstochen werden kann. Bei dieser Ausführungsform wird dabei für das Durchstechen des Bodenbereichs mehr Kraft benötigt, als zum Auseinanderziehen des Faltenbereichs. Wird die Kanüle (18) durch die Führung (12) eingeführt, wird vor dem Durchstechen des Bodenbereichs der Faltenbereich aufgefaltet, so dass wiederum dervergrößerte Eintauchstutzen als Verdrängungskörper wirkt der im Inneren des Behälters einen Überdruck aufbaut (Figur5b). Da der Überdruck nur gering ist besteht wenig Gefahr, dass der Überdruck durch Zusammendrücken des ausgezogene Faltenbalg kompensiert wird anstelle durch Verdrängen der Flüssigkeit durch das Flüssigkeitsleitsystem. Dieser Gefahr kann zusätzlich durch das gewählte Material begegnet werden.

In den Varianten nach den Figuren 4 und 5 wird derfürdas Durchstoßen notwendige Druck überz.B. die Dicke des Bodenbereichs gesteuert.

Weitere Einzelheiten, wie die Verschlusskappe oder der Behälter grundsätzlich aufgebaut sein können, können der EP 0775076 entnommen werden.

Diezuletztbeschriebenen Ausführungsformen der Erfindung kann analog in einem als auf einem Behälter sitzenden Flansch als Verschlusssystem gemäß der EP 1058657 ausgebildet sein.

## Patentansprüche

1. Verfahren zum erstmaligen Beaufschlagen eines eine Flüssigkeit leiten könnenden rohrartigen, in sich dichten Systems mit zwei offenen Enden mit einer Arzneimittelflüssigkeit, wobei das System Teil eines treibmittelfreien Inhalators ist, **dadurch gekennzeichnet, dass** auf das rohrförmige untere Ende des Systems manuell ein Behälter, der eine flüssige Arzneimittelformulierung enthält, druckdicht soweit aufgesteckt wird, dass das rohrförmige Ende in die Flüssigkeit hineinragt und ein zu Beginn oder Ende des Aufsteckungsvorgangs vorhandener Überdruck in dem Behälter von wenigstens 100 Pa (1 mbar) einen Teil der Arzneimittelflüssigkeit unter Abbau des Überdrucks so durch das System hindurch presst, dass das System vollständig mit Flüssigkeit gefüllt ist.

2. Verfahren nach Anspruch 1, **dadurch gekennzeichnet, dass** mit dem Aufsteckvorgang wenigstens ein-einhalb mal soviel Flüssigkeit durch das System gepresst wird, wie es dem Volumen des Systems entspricht.

3. Verfahren nach Anspruch 1, **dadurch gekennzeichnet, dass** der Überdruck in dem Behälter durch Kaltabfüllung der flüssigen Arzneimittelformulierung bei einer Temperatur von weniger als 10° C mit anschließender druckdichter Versiegelung des Behälters und Aufsteckung des Behälters auf das untere Ende des Hohlkolbens bei einer Temperatur von mehr als 10° C erzeugt wird.

4. Verfahren nach Anspruch 1, **dadurch gekennzeichnet, dass** der Überdruck in dem Behälter durch Abfüllung der flüssige Arzneimittelformulierung bei einem Überdruck von wenigstens 1kPa (10 mbar) unter Einschluß einer verbleibenden Luftblase von einem Volumen von wenigstens 0,1 ml bis maximal 0,5 ml und Aufsteckung des Behälters auf das untere Ende des Hohlkolbens bei Normaldruck erzeugt wird.

5. Verfahren nach Anspruch 1, **dadurch gekennzeichnet, dass** das System wenigstens einen Holkolben mit einem rohrförmigen unteren Ende und einem oberen Ende, eine Zylinderbohrung, in deren unterem Bereich der obere Bereich des Hohlkolbens zwischen zwei Positionen hin und her bewegt werden kann und eine Auslassdüse, die im oberen Ende der Zylinderbohrung angebracht ist, aufweist.

6. Verfahren nach Anspruch 1 oder 5, **dadurch gekennzeichnet, dass** das Volumen in dem Teil des System, das sich nach Eintauchen des rohrförmigen Endes oberhalb des Flüssigkeitsspiegels befindet nicht mehr als 25 Mikroliter beträgt und der Überdruck während des Aufsteckens der Kartusche auf das rohrförmige Ende dadurch erzeugt wird, dass das rohrförmige Ende des Systems soweit in die Flüssigkeit des Behälterinneren hineinragt, dass es ein Volumen von wenigstens 25 Mikroliter, stärker bevorzugt wenigstens 34 Mikroliter verdrängt.

7. Verfahren nach Anspruch 6, **dadurch gekennzeichnet, dass** das rohrförmige Ende vom unteren Bereich des Hohlkolbens nach Anspruch 5 gebildet wird.

8. Verschluss für einen mit Flüssigkeit gefüllten Behälter, der in Verschlussposition einen in den Behälter hineinragenden oder auf dem Behälter sitzenden Stutzen (2) aufweist, dessen kopfseitiges Ende von dem Behälter wegzeigt und dessen bodenseitiges Ende zum Behälterinneren ausgerichtet ist und in dem Stutzen (2) eine vom Kopfbereich beginnende rohrförmige Führung (12) ausgebildet ist, **dadurch gekennzeichnet, dass** der Stutzen (2) eine Einrichtung aufweist, die bei Einwirkung einer äußeren Kraft eine Teil der Flüssigkeit in dem Behälter verdrängt, sowie die Führung (12) an ihrem Ende einen geweiteten Bereich, bevorzugt in Form einer Kammer aufweist, die entlang der Richtung der Führung (12) zum Behälter hin geöffnet werden kann und in der sich ein Verdrängungskörper befindet, der in Richtung des Behälterinneren zumindest teilweise aus der Kammer geschoben werden kann.

9. Verschluss nach Anspruch 8, **dadurch gekennzeichnet, dass** der Verdrängungskörper eine Bohrung aufweist, die vom kopfseitigen Ende des Verdrängungskörpers beginnend ausgebildet ist in einer geraden Linie mit der Führung (12) ausgerichtet ist.

10. Verschluss nach Anspruch 9, **dadurch gekennzeichnet, dass** die Bohrung durchgängig ist und optional eine Engstelle aufweist.

11. Verschluss nach Anspruch 9, **dadurch gekennzeichnet, dass** die Bohrung nicht durchgängig ist und eine Engstellte aufweist unterhalb derer ein bodenseitig geschlossener Holraum ausgebildet ist.

12. Verschluss nach Anspruch 11, **dadurch gekennzeichnet, dass** der Verdrängungskörper als einstückiges, von Kapillaren durchzogenes, offenporig poröses Speichermedium für Flüssigkeit ausgebildet ist.

13. Verschluss nach Anspruch 12, **dadurch gekennzeichnet, dass** Verdrängungskörper ein formstabiler Körper mit einer flüssigkeitsdurchlässigen Wand, gefüllt mit gesintertem oder nicht gesintertem Pulver ist, oder ein Gewebe oder ein Gewirk oder ein Vlies oder ein Bausch aus Fasern ist.

14. Verschluss nach Anspruch 12, **dadurch gekennzeichnet, dass** das Speichermedium für Flüssigkeit in dem Verdrängungskörper aus Kunststoff, Keramik, Glas, Metall oder aus einem Naturstoff besteht.

15. Verschluss nach einem der Ansprüche 8 bis 14, **dadurch gekennzeichnet, dass** am Verdrängungskörper (14) und an der Führung (12) jeweils Stoppmittel angebracht sind, die verhindern, dass der Verdrängungskörper (14) vollständig die Führung (12) verlassen kann.

16. Verschluss nach einem der Ansprüche 8 bis 15, **dadurch gekennzeichnet, dass** die Wandung des Verdrängungskörpers (14) und die Wandung der Führung (12) flüssigkeitsdicht wechselwirken.

17. Verschluss nach Anspruch 8, **dadurch gekennzeichnet, dass** der Stutzen bodenseitig wenigstens zwei teleskopartig ineinander gesteckte Hülsen aufweist, von denen zumindest der hole Bereich der innersten Hülse in direkter Linie mit der Führung (12) ausgerichtet ist.

18. Verschluss nach Anspruch 17, **dadurch gekennzeichnet, dass** der Außendurchmesser des oberen Bereichs einer jeweils inneren Hülse größer ist als der Innendurchmesser des Fußbereichs der sie umgebenden äußeren Hülse.

19. Verschluss nach einem der Ansprüche 17 oder 18, **dadurch gekennzeichnet, dass** der innerste Durchmesser der innersten Hülse als Presspassung für eine Kanüle ausgebildet ist.

20. Verschluss nach einem der Anspruch 17, **dadurch gekennzeichnet, dass** zumindest die bodenseitige Wandung des Stutzen als Faltenbalg aus einem elastischen Material ausgebildet ist.

21. Verschluss nach einem der vorangegangenen Ansprüche 8 bis 20, **dadurch gekennzeichnet, dass** der Stutzen (2) ein nicht abdichtender Eintauchstutzen (2) ist, der während des Aufschiebens der Verschlusses (1) auf den Hals des Behälters (3) einen Teil des Behälterinhalts verdrängt.

22. Verschluss nach Anspruch 21, **dadurch gekennzeichnet, dass** eine oder mehrere Entlüftungsöffnung(en) (6) so an der Außenseite der Verschlusses (1) angeordnet ist (sind), daß diese während des Verschließens des Behälters bis zum Einrasten der innenliegenden am unteren Rand des Verschlusses umlaufenden Crimpkante (4) in die Verschlußposition mindestens eine Verbindung zwischen dem von dem Verschluss und dem Hals des Behälters gebildeten Verschlußinnenraum (7) und der Außenseite herstellt (herstellen).

23. Verschluss nach einem der vorangegangenen Ansprüche 8 bis 20, **dadurch gekennzeichnet, dass** der Stutzen (2) Teil eines Flansches ist, der auf einem Behälter sitzt.

24. System aus Verschluss nach einem der Ansprüche 8 bis 23 und einem mit Flüssigkeit gefüllten Behälter, dessen einzige Verbindung zur Außenumgebung durch den Verschluss geschlossen ist.

25. Verwendung eines Systems nach Anspruch 24 als Vorratssystem für eine flüssige Arzneimittelformulierung in einem Inhalator, der eine Kanüle (18) aufweist, die an ihrem einem Ende mit einer Düse verbunden ist und auf deren anderes Ende das System druck- und flüssigkeitsdicht aufgesteckt werden kann.

26. Verwendung eines Systems nach Anspruch 25, **dadurch gekennzeichnet, dass** die Kanüle (18) komplementär zu der Führung (12) ausgebildet ist, so dass die Kanüle (18) die Führung (12) ganz oder teilweise durchdringen kann und/oder kraftschlüssig mit der Wandung der Führung (12) wechselwirken kann.

## Claims

1. Method for applying a pharmaceutical fluid, for the first time, to an inherently sealed tubular system capable of conveying fluid, having two open ends, the system being part of a propellant-free inhaler, **characterised in that** a container, which contains a fluid pharmaceutical formulation, is pushed manually onto the tubular bottom end of the system in pressuretight manner until the tubular end projects into the fluid and an excess pressure of at least 100 Pa (1 mbar) prevailing in the container at the start or finish of the pushing-on operation forces some of the pharmaceutical fluid through the system, thereby reducing the excess pressure, such that the system is totally filled with fluid.

2. Method according to claim 1, **characterised in that** by the pushing-on process at least one and a half times as much fluid is forced through the system as corresponds to the volume of the system.

3. Method according to claim 1, **characterised in that** the excess pressure in the container is generated by cold-filling the fluid pharmaceutical formulation at a temperature of less than 10°C followed by pressuretight sealing of the container and pushing the container onto the bottom end of the hollow piston at a temperature of more than 10°C.

4. Method according to claim 1, **characterised in that** the excess pressure in the container is generated by filling the fluid pharmaceutical formulation at an excess pressure of at least 1 kPa (10 mbar) with the inclusion of a residual air bubble with a volume of at least 0.1 ml to a maximum of 0.5 ml and pushing the container onto the bottom end of the hollow piston at normal pressure.

5. Method according to claim 1, **characterised in that** the system comprises at least one hollow piston having a tubular lower end and an upper end, a cylinder bore in the lower part of which the upper region of the hollow piston can be moved back and forth between two positions and an outlet nozzle which is provided at the upper end of the cylinder bore.

6. Method according to claim 1 or 5, **characterised in that** the volume **in that** part of the system which is above the fluid level after the immersion of the tubular end is not more than 25 micro litres and the excess pressure while the cartridge is being pushed onto the tubular end is generated by the fact that the tubular end of the system projects so far into the fluid inside the container that it displaces a volume of at least 25 microlitres, more preferably at least 34 microlitres.

7. Method according to claim 6, **characterised in that** the tubular end is formed by the lower part of the hollow piston according to claim 5.

8. Closure for a fluid-filled container which comprises, in the closed position, a connector (2) projecting into the container or located on the container, the top end of which points away from the container and the bottom end of which is aligned with the interior of the container and a tubular guide (12) starting from the top part is formed in the connector (2), **characterised in that** the connector (2) has a device which displaces some of the fluid in the container under the effect of an external force, and the guide (12) comprises at its end an expanded portion, preferably in the form of a chamber, which can be opened toward the container along the direction of the guide (12) and in which there is a displacement member which can be pushed at least partially out of the chamber in the direction of the interior of the container.

9. Closure according to claim 8, **characterised in that** the displacement member has a bore, which is constructed starting from the top end of the displacement member, is aligned in a straight line with the guide (12).

10. Closure according to claim 9, **characterised in that** the bore passes right through and optionally has a constriction.

11. Closure according to claim 9, **characterised in that** the bore does not pass right through and has a constriction underneath which there is a hollow space closed off at the bottom.

12. Closure according to claim 11, **characterised in that** the displacement member is constructed as an integral, capillaried, open-pored porous storage medium for fluid.

13. Closure according to claim 12, **characterised in that** the displacement member is a dimensionally rigid body having a fluid-pervious wall, filled with sintered or non-sintered powder, or a woven or knitted or nonwoven structure or a wad of fibres.

14. Closure according to claim 12, **characterised in that** the storage medium for fluid in the displacement member consists of plastics, ceramics, glass, metal or a natural substance.

15. Closure according to one of claims 8 to 14, **characterised in that** stop means are provided on the displacement member (14) and on the guide (12), to prevent the displacement member (14) from being able to leave the guide (12) completely.

16. Closure according to one of claims 8 to 15, **characterised in that** the wall of the displacement member (14) and the wall of the guide (12) cooperate in fluidtight manner.

17. Closure according to claim 8, **characterised in that** the connector comprises at its bottom end at least two sleeves inserted telescopically one inside the other, of which at least the hollow part of the innermost sleeve is aligned directly with the guide (12).

18. Closure according to claim 17, **characterised in that** the external diameter of the upper part of a respective inner sleeve is greater than the internal diameter of the bottom part of the outer sleeve enclosing it.

19. Closure according to one of claims 17 or 18, **characterised in that** the innermost diameter of the innermost sleeve is constructed as a press fit for a cannula.

20. Closure according to one of claim 17, **characterised in that** at least the bottom wall of the connector is constructed as a bellows made of an elastic material.

21. Closure according to one of the preceding claims 8 to 20, **characterised in that** the connector (2) is a non-sealing immersion connector (2), which displaces some of the contents of the container while the closure (1) is being pushed onto the neck of the container (3).

22. Closure according to claim 21, **characterised in that** one or more vent opening(s) (6) is (are) provided on the outside of the closure (1) such that, during the closing of the container to the point where the crimp edge (4) which runs around the inside of the lower edge of the closure engages in the closure position, it (they) create(s) at least one connection between the closure interior (7) formed by the closure and the neck of the container, and the exterior.

23. Closure according to one of the preceding claims 8 to 20, **characterised in that** the connector (2) is part of a flange which is located on a container.

24. System comprising a closure according to one of claims 8 to 23 and a fluid-filled container, whose sole connection to the outer environment is sealed off by the closure.

25. Use of a system according to claim 24 as a supply system for a liquid pharmaceutical formulation in an inhaler, which comprises a cannula (18), which is connected at one end to a nozzle and at its other end the system may be pushed on in pressure- and fluid-tight manner.

26. Use of a system according to claim 25, **characterised in that** the cannula (18) is constructed to be complementary to the guide (12), so that the cannula (18) is able to pass through the guide (12) wholly or partially and/or can cooperate by frictional engagement with the wall of the guide (12).

## Revendications

1. Procédé de remplissage initial d'un système tubulaire étanche en soi pouvant faire passer un liquide, doté de deux extrémités ouvertes avec un liquide médicamenteux, le système faisant partie d'un inhalateur dépourvu d'agent propulseur, **caractérisé en ce qu'**un récipient contenant une formulation médicamenteuse liquide est monté manuellement, de façon étanche à la pression, sur l'extrémité tubulaire inférieure du système de façon à ce que l'extrémité tubulaire fasse saillie vers l'intérieur dans le liquide et qu'une surpression existante au début ou à la fin du processus de fixation dans le récipient d'au moins 100Pa (1 mbar) presse une partie du liquide médicamenteux à travers le système sous l'effet du relâchement de la surpression, de telle sorte que le système est complètement rempli de liquide.

2. Procédé selon la revendication 1, caractérisé en ce l'opération de fixation, presse à travers le système un volume de liquide égal à au moins une fois et demi le volume du système.

3. Procédé selon la revendication 1, **caractérisé en ce que** la surpression dans le récipient est générée par le remplissage à froid de la formulation médicamenteuse liquide à une température inférieure à 10°C, suivi du scellement étanche à la pression du récipient et de la fixation du récipient sur l'extrémité inférieure du piston creux à une température supérieure à 10°C.

4. Procédé selon la revendication 1, **caractérisé en ce que** la surpression dans le récipient est générée par le remplissage avec la formulation médicamenteuse liquide à une surpression d'au moins 1 kPa (10 mbar), sous l'inclusion d'une bulle résiduelle d'un volume d'au moins 0,1 ml jusqu'à au maximum 0,5 ml, et par la fixation du récipient sur l'extrémité inférieure du piston creux à pression normale.

5. Procédé selon la revendication 1, **caractérisé en ce que** le système comprend au moins un piston creux doté d'une extrémité inférieure tubulaire et d'une extrémité supérieure, d'un alésage de cylindre, dans le secteur inférieur duquel la section supérieure du piston creux peut osciller entre deux positions et une buse d'échappement qui est montée dans l'extrémité supérieure de l'alésage du cylindre.

6. Procédé selon la revendication 1 ou 5, **caractérisé en ce que** le volume dans la partie du système qui se trouve après l'immersion de l'extrémité tubulaire au-dessus du niveau du liquide ne dépasse plus 25 microlitres et **en ce que** la surpression au cours de la fixation de la cartouche sur l'extrémité tubulaire est générée par le fait que l'extrémité tubulaire du système fasse saillie dans le liquide à l'intérieur du récipient jusqu'à ce qu'un volume d'au moins 25 microlitres, de préférence d'au moins 34 microlitres soit refoulé.

7. Procédé selon la revendication 6, **caractérisé en ce que** l'extrémité tubulaire du secteur inférieur du piston creux est conçue selon la revendication 5.

8. Dispositif d'obturation pour un récipient rempli de liquide qui comprend, en position de fermeture, un embout (2) faisant saillie dans le récipient ou reposant sur le récipient dont l'extrémité située du côté de la tête est dirigée à l'opposé du récipient et son extrémité inférieure est orientée vers l'intérieur du récipient et une conduite (12) tubulaire commençant à partir de la zone de tête est formée dans l'embout (2), **caractérisé en ce que** l'embout (2) comprend un dispositif qui, sous l'action d'une force extérieure refoule une partie du liquide dans le récipient, et la conduite (12) comprend à son extrémité un secteur élargi, de préférence sous la forme d'une chambre qui peut être ouverte le long de la direction de la conduite (12) vers le récipient et dans laquelle se trouve un corps de refoulement qui peut être poussé au moins en partie hors de la chambre en direction de l'intérieur du récipient.

9. Dispositif d'obturation selon la revendication 8, **caractérisé en ce que** le corps de refoulement comprend un alésage qui est conçu en prenant son origine à partir de l'extrémité située du côté tête du corps de refoulement et qui est aligné avec la conduite (12) suivant une ligne droite.

10. Dispositif d'obturation selon la revendication 9, **caractérisé en ce que** l'alésage est traversant et possède en option un goulot d'étranglement.

11. Dispositif d'obturation selon la revendication 9, **caractérisé en ce que** l'alésage n'est pas traversant et comprend un goulot d'étranglement, au-dessous duquel est formée une cavité fermée du côté du fond.

12. Dispositif d'obturation selon la revendication 11, **caractérisé en ce que** le corps de refoulement est conçu sous forme de support de stockage de liquide, d'un seul tenant, poreux, à pores ouverts, traversé par des conduits capillaires.

13. Dispositif d'obturation selon la revendication 12, **caractérisé en ce que** le corps de refoulement est un corps indéformable doté d'une paroi perméable au liquide, rempli d'une poudre frittée ou non frittée, ou un tissu ou un maillage ou un non tissé ou un tampon à base de fibres.

14. Dispositif d'obturation selon la revendication 12, **caractérisé en ce que** le support de stockage de liquide dans le corps de refoulement est en plastique, céramique, verre, métal ou en une matière naturelle.

15. Dispositif d'obturation selon l'une quelconque des revendications 8 à 14, **caractérisé en ce que** des dispositifs d'arrêt sont montés respectivement au niveau du corps de refoulement (14) et au niveau de la conduite (12), qui empêchent que le corps de refoulement (14) ne sorte complètement de la conduite (12).

16. Dispositif d'obturation selon l'une quelconque des revendications 8 à 15, **caractérisé en ce que** la paroi du corps de refoulement (14) et la paroi de la conduite (12) interagissent de façon étanche aux liquides.

17. Dispositif d'obturation selon la revendication 8, **caractérisé en ce que** l'embout comprend du côté du fond au moins deux manchons emboîtés l'un dans l'autre de façon télescopique, dont au moins le secteur creux du manchon le plus interne est directement aligné avec la conduite (12).

18. Dispositif d'obturation selon la revendication 17, **caractérisé en ce que** le diamètre extérieur du secteur supérieur d'un manchon interne est plus grand que le diamètre intérieur du secteur inférieur du manchon extérieur l'entourant.

19. Dispositif d'obturation selon l'une quelconque des revendications 17 ou 18, **caractérisé en ce que** le diamètre le plus interne du manchon le plus interne est conçu sous forme d'ajustement serré pour une canule.

20. Dispositif d'obturation selon la revendication 17, **caractérisé en ce qu'**au moins la paroi inférieure de l'embout est conçue sous forme de soufflet en matériau élastique.

21. Dispositif d'obturation selon l'une des revendications précédentes 8 à 20, **caractérisé en ce que** l'embout (2) est un embout d'immersion (2) non étanche qui, lorsque le dispositif d'obturation (1) est poussé sur le col du récipient (3), refoule une partie du contenu du récipient.

22. Dispositif d'obturation selon la revendication 21, **caractérisé en ce qu'**un ou plusieurs orifice(s) de purge d'air (6) est (sont) disposé(s) sur la face externe du dispositif d'obturation (1) de telle sorte que, lors de la fermeture du récipient jusqu'à ce que le bord de sertissage circulaire situé à l'intérieur sur le bord inférieur du dispositif d'obturation vienne s'encliqueter en position de fermeture, au moins un assemblage est produit entre l'intérieur du dispositif d'obturation (7), formé par le dispositif d'obturation et le col du récipient, et l'extérieur.

23. Dispositif d'obturation selon l'une des revendications précédentes 8 à 20, **caractérisé en ce que** l'embout (2) fait partie d'un rebord qui est situé sur un récipient.

24. Système composé d'un dispositif d'obturation selon l'une des revendications précédentes 8 à 23 et d'un récipient rempli de liquide dont le seul lien avec l'environnement extérieur est fermé par le dispositif d'obturation.

25. Utilisation d'un système selon la revendication 24 en tant que système de stockage pour une formulation médicamenteuse liquide dans un inhalateur qui comprend une canule (18) qui est reliée, à une de ses extrémités, à une buse et sur l'autre extrémité de laquelle le système peut être relié de façon étanche aux liquides et à la pression.

26. Utilisation d'un système selon la revendication 25, **caractérisé en ce que** la canule (18) est conçue de façon à être complémentaire à la conduite (12), de sorte que la canule (18) peut pénétrer entièrement ou en partie la conduite (12) et/ou peut interagir par liaison de force avec la paroi de la conduite (12).
